# FASCICULE DE BREVET EUROPEEN

(11) **EP 3 134 138 B1**
(45) Date de publication et mention de la délivrance du brevet: **11.04.2018**
(21) Numéro de dépôt: 15725770.0
(22) Date de dépôt: 21.04.2015
(51) Int. Cl.: A61L 27/36, A61L 27/46

(54) **PROCÉDÉ DE FABRICATION DE PÂTE OSSEUSE**
VERFAHREN ZUR HERSTELLUNG EINER KNOCHENPASTE
METHOD FOR PRODUCING A BONE PASTE

(30) Priorité: 25.04.2014 FR 1453726
(43) Date de publication de la demande: 01.03.2017
(73) Titulaire: Biobank, 77220 Presles en Brie (FR)
(72) Inventeur: BARDONNET, Raphaël, 77240 Seine-Port (FR); BARBEITO, Ana, 75013 Paris (FR)
(74) Mandataire: Cabinet Plasseraud
(86) Numéro de dépôt international: PCT/FR2015/051076
(87) Numéro de publication internationale: WO 2015/162372

(56) Documents cités:
- WO-A2-99/38543
- US-A1- 2007 202 190

## Description

### DOMAINE DE L'INVENTION

L'invention concerne les procédés de préparation de pâte osseuse.

### ARRIERE-PLAN DE L'INVENTION

Le document EP1341545 décrit un exemple de procédé de ce type, dans lequel on chauffe un mélange d'os déminéralisé et d'eau pour obtenir un support pâteux (phase visqueuse), que l'on mélange ensuite avec de l'os déminéralisé ou non déminéralisé (phase solide) pour former un gel osseux ou un mastic osseux. Ce procédé est complexe puisqu'il présente tout d'abord une étape de préparation de la phase visqueuse à laquelle se rajoute secondairement l'addition de la phase solide. Le mélange doit être réalisé en environnement stérile pour une utilisation chirurgicale, ce qui renforce sa complexité. De plus, ce procédé présente notamment les inconvénients que la pâte osseuse ainsi obtenue est généralement inhomogène puisque les deux phases ne présentent pas de continuité.

On connaît aussi, par le document US 2007/0202190, un procédé similaire qui utilise comme matière première l'os cortical. D'une part une fraction de cette matière première est soumise un traitement thermique à moins de 100°C (sans atteindre ni a *fortiori* dépasser une température d'ébullition) et acide pour extraire de la gélatine et constituer ainsi, après neutralisation et séchage, une gélatine minéralisée. D'autre part une autre fraction de cette matière première est déminéralisée de façon appropriée (typiquement par un traitement acide suivi d'une neutralisation et d'un séchage), pour constituer de la poudre d'os déminéralisée appelée DBM (Demineralized Bone Matrix). La pâte osseuse obtenue par ce procédé résulte du mélange de la gélatine minéralisée, de la DBM, et d'une phase liquide, de sorte qu'un effet ostéo-inducteur est obtenu. La question de l'injectabilité, typiquement par une seringue, se pose avec ce type de mélange du fait de la présence de grains de constitution différente. De plus, aucun procédé de stérilisation terminale ne pouvant être appliqué au mélange final, la stérilité du produit (nécessaire à un usage thérapeutique) oblige à travailler en conditions stériles, ce qui rend le procédé coûteux et complexe.

D'autre part, la préservation des protéines ostéoinductrices de la fraction DBM est un facteur de risque sur le plan sanitaire puisque les prions, qui sont des protéines, ne sont pas éliminés lors de sa préparation et risquent d'être transmis au patient receveur. C'est pourquoi le procédé prévu par le document US 2007/0202190, complexe avec des étapes délicates, a un usage en pratique limité.

Par ailleurs, le document US-2002-0076429 décrit un procédé consistant à obtenir un support pâteux (phase visqueuse) à partir de la gélatine déshydratée qui a subi un traitement thermique (par exemple par autoclavage) pour à la fois stériliser et augmenter la viscosité de celle-ci. Cette gélatine est ensuite broyée stérilement et mélangée à un produit ostéoconducteur et/ou ostéoinducteur. Le procédé complet implique que le broyage puis le mélange avec le produit ostéoconducteur et/ou ostéoinducteur soient faits en environnement stérile, ce qui rend le procédé complexe et coûteux. Par ailleurs, le produit obtenu est solide à 37°C, et oblige le chirurgien à chauffer le produit pour le ramollir avant de l'implanter, ce qui rend le produit complexe à mettre en oeuvre.

### OBJETS ET RESUME DE L'INVENTION

La présente invention a notamment pour but de pallier tout ou partie de ces inconvénients.

A cet effet, l'invention propose un procédé pour préparer une pâte osseuse ostéoconductrice, comprenant au moins les étapes suivantes :
(a) une étape de fourniture au cours de laquelle on fournit, dans un contenant, un mélange de départ contenant une solution aqueuse et un produit granulaire comprenant au moins du collagène et des minéraux, le collagène représentant entre 30 % et 90 % en poids sec du produit granulaire et les minéraux représentant entre 10 % et 70 % en poids sec du produit granulaire, au moins une partie du collagène étant contenu dans des grains de matrice osseuse au moins partiellement minéralisée,
(b) une étape de chauffage au cours de laquelle on chauffe, dans le contenant, le mélange de départ à une température d'au moins 100°C, de préférence 121 °C, pour transformer en gélatine une partie du collagène, de sorte que l'on établit une continuité entre la gélatine et la matrice osseuse au moins partiellement minéralisée et que l'on obtient ainsi une pâte osseuse cohésive, l'étape de chauffage permettant la stérilisation de la pâte osseuse dans le contenant,
ce grâce à quoi après refroidissement et gélification, la pâte osseuse est prête à l'emploi et maintenue stérile dans le contenant dans un état de fermeture étanche du contenant.

Grâce à ces dispositions, on obtient une pâte osseuse homogène et prête à l'emploi, dont la composition mixte (au moins gélatine/matrice osseuse déminéralisée/matrice osseuse minéralisée) est favorable à l'ostéoconduction, et lui confère de bonnes propriétés de malléabilité (la pâte osseuse obtenue est déformable plastiquement) et de cohésion (notamment, cette pâte osseuse ne fond pas à 38 °C). Ces propriétés particulièrement avantageuses sont obtenues du fait que le chauffage est appliqué au mélange complet (en une étape), permettant ainsi d'obtenir une excellente continuité entre la phase solide et la phase visqueuse (gélatine).

La cuisson à une température autour de 121 °C, ou plus généralement supérieure à 100°C, permet de transformer les composants du produit granulaire (en particulier avec exsudation de gélatine issue des grains, en particulier ceux déjà partiellement ou totalement déminéralisés), tout en ayant un effet de stérilisation avantageux. Ainsi, les grains de matrice osseuse au moins partiellement minéralisée sont aussi transformés du fait du chauffage, avec un bénéfice pour la rhéologie de la pâte osseuse. Une continuité de phases entre la gélatine, la fraction déminéralisée et la fraction minéralisée est obtenue. On comprend qu'il n'est pas nécessaire, pour obtenir le caractère pâteux, d'ajouter un quelconque liant au cours d'une étape de mélange qui serait nuisible au maintien de l'état stérile.

La pâte osseuse ainsi obtenue de façon fiable et simple est ostéoconductrice, stérile et prête à l'emploi. Le mélange étant soumis intégralement à un chauffage à plus de 100 °C, typiquement par autoclavage, on comprend que la préservation de protéines ostéoinductrices n'est pas recherchée, avec avant tout le bénéfice de la sécurité virale et de la stérilité du produit fini. Le produit fini peut être préparé dans un récipient étanche, de sorte que sa composition est parfaitement maîtrisée et il peut être directement injecté si le récipient étanche est une seringue.

On notera que dans la présente demande, le terme « matrice osseuse minéralisée » est utilisé pour désigner une matrice osseuse (tissu osseux) qui n'a subi aucun traitement de déminéralisation et qui comprend une composante minérale (environ 70 % en poids sec) et une composante collagénique (environ 30 % en poids sec). Cette matrice osseuse peut être d'origine humaine ou animale et peut être de l'os cortical, spongieux ou cortico-spongieux.

On notera que dans la présente demande, les termes tels que « granulaire » ou « grain » sont utilisés pour désigner tout matériau formé de particules solides, de fragments ou de filaments de taille comprise entre 0 et quelques millimètres.

Dans divers modes de réalisation du procédé selon l'invention, on peut éventuellement avoir recours en outre à l'une et/ou à l'autre des dispositions suivantes :
- à l'étape de chauffage (b), le mélange de départ est chauffé pendant une durée comprise entre 10 mn et 2 h, préférentiellement comprise entre 18 mn et 1 h ;
- à l'étape de chauffage (b), le mélange de départ est chauffé à une température comprise entre 50 °C et 200 °C, par exemple entre 100 °C et 200 °C, préférentiellement comprise entre 100 °C et 150 °C ;
- à l'étape de chauffage (b) le mélange de départ est chauffé pendant une durée comprise entre 18 mn et 1 h et à une température comprise entre 100°C et 150 °C ;
- à l'étape de fourniture (a), le mélange de départ est fourni dans un contenant dans un état de fermeture étanche et il est chauffé à l'étape de chauffage (b) dans ledit contenant dans cet état de fermeture étanche ; le terme étanche signifie ici hermétique à la vapeur d'eau et aux microorganismes jusqu'à une pression différentielle de au moins 1 bar (on utilisera dans ce qui suit l'expression plus concise « contenant étanche ») ;
- à l'étape de fourniture (a), le contenant étanche est mis dans un sachet scellé et il est chauffé à l'étape de chauffage (b) dans ledit sachet scellé : on obtient ainsi une pâte osseuse stérile entièrement conditionnée, prête à l'emploi ;
- l'étape de chauffage (b) est réalisée à une température suffisante et pendant une durée suffisante pour stériliser la pâte osseuse ;
- l'étape de chauffage (b) est réalisée à la vapeur d'eau, au bain marie, ou à la chaleur sèche ;
- à l'étape de fourniture (a), les minéraux représentent entre 20 % et 60 % en poids sec du produit granulaire et le collagène représente entre 40 et 80 % en poids sec du produit granulaire ;
- à l'étape de fourniture (a), la solution aqueuse du mélange de départ est choisie parmi l'eau, un sérum physiologique ou une solution de tampon physiologique ;
- à l'étape de fourniture (a), la solution aqueuse représente entre 40 % et 75 % en poids total du mélange de départ ;
- à l'étape de fourniture (a), le matériau granulaire a une granulométrie inférieure à 4 mm, préférentiellement inférieure à 1,5 mm ;
- à l'étape de fourniture (a), le mélange de départ comporte en outre un biomatériau ostéoconducteur minéralisé ; ce biomatériau ostéoconducteur minéralisé peut être notamment d'origine humaine ou animale (matrice osseuse déprotéinée) ou synthétique (hydroxyapatite, phosphate tricalcique, bio-verre ou autre matériau implantable) ;
- à l'étape de fourniture (a), le mélange de départ comporte des grains de matrice osseuse totalement déminéralisée comprenant principalement du collagène et des grains de matrice osseuse minéralisée comprenant du collagène et des minéraux ;
- à l'étape de fourniture (a), le mélange de départ comporte des grains de matrice osseuse partiellement déminéralisée comprenant entre 20 % et 70 % de minéraux en poids sec, préférentiellement entre 40 % et 60 % de minéraux en poids sec ;
- à l'étape de fourniture (a), le mélange de départ comprend du collagène purifié et des grains de matrice osseuse minéralisée ;
- l'étape de fourniture (a) comprend au moins les sous-étapes suivantes :
   (a1) une sous-étape de préparation de grains de matrice osseuse totalement déminéralisée par déminéralisation et broyage à partir d'os (la déminéralisation précédant le broyage ou lui succédant),
   (a2) une sous-étape de préparation de grains de matrice osseuse minéralisée par broyage d'os,
   (a3) une sous-étape de mélangeage au cours de laquelle on mélange au moins les grains de matrice osseuse totalement déminéralisée, les grains de matrice osseuse minéralisée et la solution aqueuse pour obtenir ledit mélange de départ ;
- l'étape de fourniture (a) comprend au moins les sous-étapes suivantes :
   (a'1) une sous-étape de préparation de grains de matrice osseuse partiellement déminéralisée par déminéralisation et broyage d'os (la déminéralisation précédant le broyage ou lui succédant),
   (a'3) une sous-étape de mélangeage au cours de laquelle on mélange au moins les grains de matrice osseuse partiellement déminéralisée et la solution aqueuse pour obtenir ledit mélange de départ ;
- l'étape de fourniture (a) comprend au moins les sous-étapes suivantes :
   (a"2) une sous-étape de préparation de grains de matrice osseuse minéralisée par broyage d'os,
   (a"3) une sous-étape de mélangeage au cours de laquelle on mélange au moins les grains de matrice osseuse minéralisée, le collagène purifié et la solution aqueuse pour obtenir ledit mélange de départ.
- au cours de la sous-étape de mélangeage (a3, a'3 ou a"3), on mélange un biomatériau ostéoconducteur minéralisé avec les autres constituants du mélange de départ ;
- au cours de la sous-étape de mélangeage (a3, a'3 ou a"3), on mélange au moins de l'acide hyaluronique et/ou ses dérivés, en particulier l'acide hyaluronique réticulé, avec les autres constituants du mélange de départ.

### BREVE DESCRIPTION DES DESSINS

D'autres caractéristiques et avantages de l'invention apparaîtront au cours de la description suivante d'une de ses modes de réalisation, donné à titre d'exemple non limitatif, en regard du dessin joint.

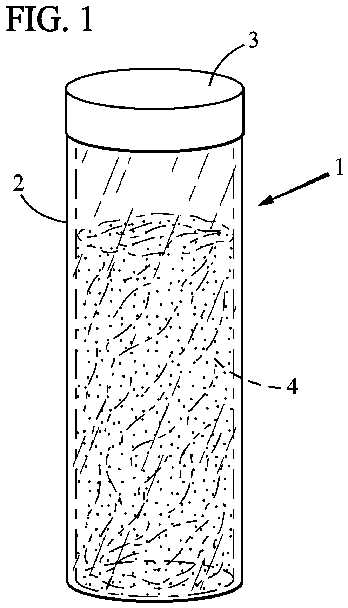

Sur les dessins, la figure 1 représente un flacon contenant de la pâte osseuse obtenue par le procédé selon l'invention.

La figure 2 est une photo montrant l'aspect de la pâte osseuse issue du procédé conformément au mode de préparation préféré.

La figure 3 est une photo montrant l'aspect de grains d'une poudre d'os brute, avant mise en oeuvre du procédé.

### DESCRIPTION PLUS DETAILLEE

Le procédé selon l'invention permet de préparer une pâte osseuse malléable qui peut être utilisée comme produit de comblement osseux dans les applications médicales.

Cette pâte osseuse peut être préparée à partir d'une fraction collagénique et d'une fraction minéralisée. La fraction collagénique est issue d'une matrice osseuse au moins partiellement déminéralisée ou de collagène purifié. La fraction minéralisée est issue de tissu osseux d'origine humaine ou animale, de type cortical, spongieux ou cortico-spongieux appelé ici matrice osseuse minéralisée.

Le procédé de préparation de pâte osseuse selon l'invention, comporte notamment deux grandes étapes :
(a) fourniture d'un mélange de départ contenant une solution aqueuse, une fraction collagénique et une fraction minéralisée,
(b) chauffage.

### (a) Etape de fourniture du mélange de départ :

Au cours de cette étape (a), on fournit un mélange de départ contenant au moins :
- une solution aqueuse, et
- un produit granulaire comprenant au moins du collagène et des minéraux, au moins une partie du collagène étant contenu dans des grains de matrice osseuse au moins partiellement minéralisée.

Le collagène peut provenir d'une matrice osseuse totalement ou partiellement déminéralisée ou de toute autre source de collagène purifié.

Le mélange de départ peut par exemple être constitué comme suit :
- il peut comprendre des grains de matrice osseuse totalement déminéralisée ou de collagène purifié et des grains de matrice osseuse minéralisée ;
- il peut comprendre des grains de matrice osseuse partiellement déminéralisée.

Dans le produit granulaire du mélange de départ, le collagène peut représenter entre 30 % et 90 % en poids sec du produit granulaire (avantageusement entre 40 % et 80 %) et les minéraux entre 10 % et 70 % en poids sec du produit granulaire (avantageusement entre 20 % et 60 %).

Le produit granulaire en question constitue de préférence un mélange homogène.

Les grains qui constituent le mélange de départ peuvent avoir une granulométrie inférieure à 4 mm, préférentiellement inférieure à 1,5 mm.

La solution aqueuse peut représenter entre 40 % et 75 % en poids total du mélange. Selon la proportion en eau, la viscosité de la pâte osseuse finale peut être ajustée en fonction des besoins. Une proportion d'eau diminuée jusqu'à 40 % donnera une viscosité plus élevée, pour atteindre une consistance de mastic. Une proportion d'eau augmentée jusqu'à 75 % donnera une viscosité moins élevée, pour atteindre une consistance de gel. Cette observation est valable pour toutes les formes de réalisation de l'invention.

La solution aqueuse du mélange de départ peut être choisie notamment parmi l'eau, un sérum physiologique (solution de chlorure de sodium, par exemple à 0,9 %) ou une solution tampon physiologique de phosphate ou autre.

Le produit granulaire du mélange de départ peut éventuellement comporter en outre un biomatériau ostéoconducteur minéralisé ; ce biomatériau ostéoconducteur minéralisé peut être notamment d'origine humaine ou animale (matrice osseuse déprotéinée) ou synthétique (hydroxyapatite, phosphate tricalcique, bio-verre ou autre matériau implantable). Le biomatériau minéralisé peut avoir une granulométrie inférieure à 4 mm, préférentiellement inférieure à 1,5 mm. La proportion de biomatériau minéralisé éventuellement ajouté au mélange de départ peut représenter entre 10 % et 40 % du poids sec du mélange de départ. Plus généralement, la proportion de biomatériau ostéoconducteur additionnel peut être ajustée pour maintenir à la pâte osseuse finale ses propriétés de malléabilité et de cohésion.

Plusieurs cas de préparation du mélange initial vont être détaillés ci-dessous.

### 1. Cas où le mélange de départ comprend des grains de matrice osseuse minéralisée et des grains de matrice osseuse totalement déminéralisée :

Lorsque le mélange de départ comprend des grains de matrice osseuse minéralisée et des grains de matrice osseuse totalement déminéralisée, l'étape (a) de fourniture du mélange de départ peut comprendre au moins les sous-étapes suivantes :
(a1) une sous-étape de préparation de grains de matrice osseuse totalement déminéralisée par déminéralisation et broyage à partir de fragments d'os (la déminéralisation précédant le broyage ou lui succédant) ;
(a2) une sous-étape de préparation de grains de matrice osseuse minéralisée par broyage de fragments d'os ;
(a3) une sous-étape de mélangeage au cours de laquelle on mélange les grains de matrice osseuse totalement déminéralisée, les grains de matrice osseuse minéralisée et la solution aqueuse pour obtenir ledit mélange de départ.

### 2. Cas où le mélange de départ comprend des grains de matrice osseuse partiellement déminéralisée :

Lorsque le mélange de départ comprend des grains de matrice osseuse partiellement déminéralisée, l'étape (a) de fourniture du mélange de départ peut comprendre au moins les sous-étapes suivantes :
(a'1) une sous-étape de préparation de grains de matrice osseuse partiellement déminéralisée par déminéralisation et broyage de fragments d'os (la déminéralisation précédant le broyage ou lui succédant) ;
(a'3) une sous-étape de mélangeage au cours de laquelle on mélange les grains de matrice osseuse partiellement déminéralisée et la solution aqueuse pour obtenir ledit mélange.

### 3. Cas où le mélange de départ comprend des grains de collagène et des grains de matrice osseuse minéralisée :

Lorsque le mélange de départ comprend des grains de collagène purifié et des grains de matrice osseuse minéralisée, l'étape (a) de fourniture du mélange de départ peut comprendre au moins les sous-étapes suivantes :
(a"1) une sous-étape de préparation de grains de collagène purifié par une technique quelconque permettant d'obtenir un collagène purifié déshydraté,
(a"2) une sous-étape de préparation de grains de matrice osseuse minéralisée par broyage de fragments d'os,
(a"3) une sous-étape de mélangeage au cours de laquelle on mélange au moins les grains de matrice osseuse, les grains de collagène purifié et la solution aqueuse pour obtenir ledit mélange de départ.

Eventuellement, au cours de la sous-étape de mélangeage (a3, a'3 ou a"3), on mélange un biomatériau ostéoconducteur minéralisé avec les autres constituants du mélange de départ.

### (b) Etape de chauffage :

Au cours de l'étape de chauffage, on chauffe le mélange de départ suffisamment pour transformer en gélatine une partie du collagène et obtenir ainsi la pâte osseuse voulue, qui est homogène et prête à l'emploi après refroidissement et gélification.

Au cours de cette étape de chauffage, le mélange de départ peut être chauffé pendant une durée comprise entre 10 mn et 2 h, préférentiellement comprise entre 18 mn et 1 h.

Le mélange de départ peut être chauffé à une température comprise entre 50 °C et 200 °C, préférentiellement comprise entre 100 °C et 150 °C.

Plus particulièrement, le mélange de départ peut avantageusement être chauffé pendant une durée comprise entre 18 mn et 1 h à une température comprise entre 100°C et 150 °C. Notamment, le mélange de départ peut avantageusement être chauffé pendant une durée de l'ordre de 30 mn à 1 h (notamment 30 mn dans le cas 1 susmentionné ou 1 h dans le cas 2 susmentionné) à une température de l'ordre de 120 °C (par exemple 121 °C).

Plus généralement, l'étape de chauffage (b) est avantageusement réalisée à une température suffisante et pendant une durée suffisante pour stériliser la pâte osseuse.

Le chauffage peut être réalisé à la vapeur d'eau, au bain marie, ou à la chaleur sèche.

Avantageusement, à la fin de l'étape de fourniture (a), le mélange de départ est mis dans un contenant étanche résistant à la chaleur et aux différences de pression dues au système de chauffage utilisé (bien évidemment le contenant à l'état fermé est rendu étanche à la vapeur d'eau de sorte qu'il peut exister des différences de pression entre l'intérieur et l'extérieur du contenant ; par exemple, le contenant résiste aux différences de pression d'au moins 1 bar lorsqu'on utilise un autoclave à 121 °C), et il est chauffé à l'étape de chauffage (b) dans ledit contenant étanche, de sorte qu'on obtient ainsi une pâte osseuse stérile prête à l'emploi après refroidissement, sans étape supplémentaire de conditionnement en récipient ou de stérilisation. Le récipient 1 en question peut être par exemple un flacon 2 tel que celui représenté en figure 1, en verre ou autre matériau supportant la température requise, fermé par un bouchon 3 vissé ou emboîté et contenant la pâte osseuse 4. Comme bien visible sur la figure 1, le bouchon 3 obture ici une unique ouverture du flacon 2. Bien entendu, l'état de fermeture étanche du contenant peut le cas échéant être obtenu au cours de l'étape de chauffage (b) ou juste après celle-ci. Et bien que la figure 1 montre un contenant en deux parties formé d'un flacon 2 et d'un bouchon 3, ceci n'est absolument pas limitatif. Le contenant peut aussi correspondre à l'utilisation d'un ensemble avec une ou plusieurs enveloppes non rigides, d'un assemblage de deux ou trois parties au moins, et/ou d'une partie à plusieurs éléments d'obturation.

Avantageusement, le contenant peut être disposé dans un double sachet scellé perméable uniquement aux gaz à la fin de l'étape (a), de sorte que le conditionnement est complet avant l'étape de chauffage (b), ce qui permet ensuite de conserver le flacon contenant 1 et la pâte osseuse 4 dans un état stérile jusqu'à utilisation au bloc opératoire (le contenant étant ouvert seulement à ce stade).

Aucune stérilisation terminale par irradiation gamma ou béta (nuisible pour l'intégrité de la gélatine) n'est nécessaire.

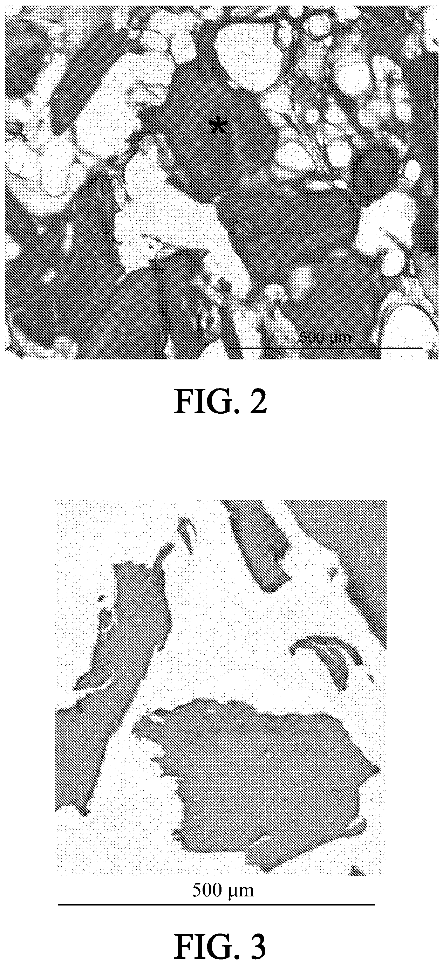

Une opération de mélange est réalisée à l'étape de fourniture (a) avec une partie liquide et une partie solide, composée de grains de matrice osseuse au moins partiellement minéralisée contenant du collagène. De tels grains peuvent être obtenus lors d'une sous-étape (préalable) de déminéralisation partielle. Sur la figure 2, une photo réalisée avec un microscope de type Leica® DM2000 relié à une caméra digitale (pilotée par un logiciel d'acquisition d'image) illustre le résultat du procédé, à l'issue de l'étape de chauffage (b). On peut voir que les grains qui ont été partiellement déminéralisés (ici tous les grains dans le cas de la figure 2) présentent une partie centrale minéralisée et une partie périphérique déminéralisée. Entre les grains, il s'est formé une phase gel par exsudation de la gélatine à partir du collagène des grains grâce au traitement spécifique avec chauffage à plus de 100 °C. Ceci permet de mieux combiner les phases de la pâte osseuse 4. A titre comparatif, une poudre d'os brute (avant traitement par le procédé, sans traitement de déminéralisation) est représentée à la figure 3. Chaque grain a une composition homogène et l'absence évidente de gel entre les grains fait que la poudre correspondante n'a pas les propriétés rhéologiques d'une pâte (non malléable, non cohésive et non injectable).

Plusieurs exemples détaillés sont donnés ci-après.

### Exemple 1 : Cas où on utilise un mélange de départ constitué de matrice osseuse totalement déminéralisée et de matrice osseuse minéralisée

### (a1) préparation des grains de matrice osseuse totalement déminéralisée :

On broie un fragment osseux cortico-spongieux sous forme de poudre, de granulométrie de préférence inférieure à 1,5 mm, les grains en question incluant les fines (granulométrie < 0,2 mm dans ce cas précis).

On déminéralise ensuite totalement les grains de matrice osseuse par deux bains successifs dans l'acide chlorhydrique 0,5 N à température ambiante pendant 4 h sous agitation à raison de 50 ml d'acide chlorhydrique par gramme de matrice osseuse (on retire ainsi 100% des minéraux de la fraction minérale). On neutralise avec une solution de soude 1 N pour ramener le pH à 7, puis on rince à l'eau et on sèche sous un courant d'air chaud pendant 15 h.

### (a2) préparation des grains de matrice osseuse minéralisée :

On broie séparément un fragment osseux cortico-spongieux minéralisé sous forme de poudre, de granulométrie de préférence inférieure à 1 mm pour constituer la matrice osseuse minéralisée.

### (a3) mélangeage :

On mélange les grains de matrice osseuse totalement déminéralisée, les grains de matrice osseuse minéralisée et l'eau. Les proportions en masse du mélange total peuvent être par exemple : matrice osseuse totalement déminéralisée : 10% ; matrice osseuse minéralisée : 25% ; eau : 65 %.

### (b) chauffage :

Le récipient contenant le mélange est chauffé par autoclavage à 121 °C pendant 30 mn.

Dans ce type de configuration, le chauffage transforme en gélatine une majeure partie des grains de matrice osseuse déminéralisée et une partie du collagène des grains de matrice osseuse minéralisée, ce qui permet d'obtenir une continuité de matière entre la phase gel (gélatine) et la phase solide (grains de matrice osseuse minéralisée). Après refroidissement et gélification, la pâte osseuse est stérile et prête à l'emploi et présente la viscosité d'un mastic.

### Exemple 2 : Cas où ou utilise un mélange de départ constitué de matrice osseuse partiellement déminéralisée, sans ajout de matrice osseuse minéralisée

### (a'1) préparation des grains de matrice osseuse partiellement déminéralisée :

On broie un fragment osseux cortico-spongieux sous forme de poudre, de granulométrie de préférence inférieure à 1,5 mm, les grains en question incluant les fines (granulométrie < 0,2 mm dans ce cas précis).

On déminéralise ensuite partiellement les grains de matrice osseuse dans l'acide chlorhydrique 0,4 N à température ambiante pendant 1 h ou 1h30 sous agitation à raison de 10 ml d'acide chlorhydrique par gramme de matrice osseuse (on retire ainsi 50 % des minéraux de la fraction minérale). On neutralise avec une solution de soude 1 N pour ramener le pH à 7, puis on rince à l'eau et on sèche sous un courant d'air chaud pendant 15 h.

### (a'3) mélangeage :

On mélange les grains de matrice osseuse partiellement déminéralisée et l'eau dans un récipient étanche. Les proportions en masse du mélange total peuvent être par exemple : grains de matrice osseuse partiellement déminéralisée 35 % ; eau : 65 %.

### (b) chauffage :

Le récipient étanche contenant le mélange est chauffé par autoclavage à 121 °C pendant 1 h.

Dans ce type de configuration, Le chauffage transforme en gélatine une partie du collagène des grains de matrice osseuse partiellement déminéralisée, ce qui permet d'obtenir une continuité de matière entre la phase gel (gélatine) et la phase solide (partie restante des grains de matrice osseuse partiellement déminéralisée). Après refroidissement et gélification, la pâte osseuse est stérile et prête à l'emploi.

Avec la proportion d'eau susmentionnée de 65 %, la pâte osseuse a une consistance de gel liquide. La viscosité de la pâte osseuse finale peut être ajustée en fonction des besoins, en modifiant cette proportion d'eau dans le mélange de départ. Par exemple, une proportion d'eau de 55 % donnera une viscosité plus élevée, tout en conservant le caractère injectable avec une seringue disposant d'un orifice d'environ 3 mm de diamètre. Cette observation est valable pour toutes les formes de réalisation de l'invention.

### Exemple 3 : Cas où on utilise un mélange de départ constitué de collagène et de matrice osseuse minéralisée

### (a"1) préparation des grains de collagène :

On sélectionne du collagène sous forme de poudre ou de filaments, déshydraté par lyophilisation ou tout autre mode de séchage.

### (a"2) préparation des grains de matrice osseuse minéralisée :

On broie séparément un fragment osseux cortico-spongieux minéralisé sous forme de poudre, de granulométrie de préférence inférieure à 1 mm pour constituer la matrice osseuse minéralisée.

### (a"3) mélangeage :

On mélange les grains de collagène, les grains de matrice osseuse minéralisée et de l'eau. Les proportions en masse du mélange total peuvent être par exemple : collagène : 10 % ; matrice osseuse minéralisée : 25 % ; eau : 65 %.

### (b) chauffage :

Le récipient contenant le mélange est chauffé par autoclavage à 121 °C pendant 30 mn.

Le chauffage transforme en gélatine une majeure partie du collagène pur et une partie du collagène des grains de matrice osseuse minéralisée, ce qui permet d'obtenir une continuité de matière entre la phase gel (gélatine) et la phase solide (grains de matrice osseuse minéralisée). Après refroidissement et gélification, la pâte osseuse est stérile et prête à l'emploi et présente la viscosité d'une pâte.

### Exemple 4 : Cas où on utilise un mélange de départ constitué de matrice osseuse partiellement déminéralisée et d'un biomatériau ostéoconducteur minéralisé

### (a'1) préparation des grains de matrice osseuse partiellement déminéralisée :

On broie un fragment osseux cortico-spongieux sous forme de poudre, de granulométrie de préférence inférieure à 1,5 mm, les grains en question incluant les fines (granulométrie < 0,2 mm dans ce cas précis).

On déminéralise ensuite partiellement les grains da matrice osseuse dans l'acide chlorhydrique 0,6 N à température ambiante pendant 1 h sous agitation à raison de 10 ml d'acide chlorhydrique par gramme de matrice osseuse (on retire ainsi 70 % des minéraux de la fraction minérale). On neutralise avec une solution de soude 1 N pour ramener le pH à 7, puis on rince à l'eau et on sèche sous un courant d'air chaud pendant 15 h.

### (a'2) fourniture d'un biomatériau ostéoconducteur minéralisé :

Le biomatériau ostéoconducteur est choisi parmi les biomatériaux synthétiques sous forme de poudre comme le phosphate tricalcique bêta. La granulométrie de la poudre de biomatériau est préférentiellement comprise entre 0,2 mm et 1 mm.

### (a'3) mélangeage :

On mélange les grains de matrice osseuse partiellement déminéralisée, la poudre de phosphate tricalcique bêta et de l'eau dans un récipient étanche. Les proportions en masse du mélange total peuvent être par exemple : grains de matrice osseuse partiellement déminéralisée : 30 % ; grains de phosphate tricalcique bêta : 10 % ; eau : 60 %.

### (b) chauffage :

Le récipient étanche contenant le mélange est chauffé par autoclavage à 121 °C pendant 45 mn. Après refroidissement et gélification, la pâte osseuse est stérile et prête à l'emploi et présente la viscosité d'un mastic.

## Revendications

1. Procédé pour préparer une pâte osseuse ostéoconductrice, comprenant au moins les étapes suivantes :
(a) une étape de fourniture au cours de laquelle on fournit, dans un contenant (2, 3), un mélange de départ contenant une solution aqueuse et un produit granulaire comprenant au moins du collagène et des minéraux, le collagène représentant entre 30 et 90 % en poids sec du produit granulaire et les minéraux représentant entre 10 et 70 % en poids sec du produit granulaire, au moins une partie du collagène étant contenu dans des grains de matrice osseuse au moins partiellement minéralisée,
(b) une étape de chauffage au cours de laquelle on chauffe, dans le contenant (2, 3), le mélange de départ à une température d'au moins 100°C, de préférence 121 °C, pour transformer en gélatine une partie du collagène, de sorte que l'on établit une continuité entre la gélatine et la matrice osseuse au moins partiellement minéralisée et que l'on obtient ainsi une pâte osseuse cohésive et de viscosité définie, l'étape de chauffage permettant la stérilisation de la pâte osseuse dans le contenant (2, 3),
ce grâce à quoi après refroidissement et gélification, la pâte osseuse (4) est prête à l'emploi et maintenue stérile dans ledit contenant (2, 3) dans un état de fermeture étanche du contenant.

2. Procédé selon la revendication 1, dans lequel à l'étape de chauffage (b), le mélange de départ est chauffé pendant une durée comprise entre 10 mn et 2 h, préférentiellement comprise entre 18 mn et 1 h.

3. Procédé selon la revendication 1 ou 2, dans lequel à l'étape de chauffage (b) le mélange de départ est chauffé pendant une durée comprise entre 18 mn et 1 h et à une température comprise entre 100 °C et 150 °C.

4. Procédé selon l'une quelconque des revendications précédentes, dans lequel à l'étape de fourniture (a), le mélange de départ est fourni dans le contenant dans un état de fermeture étanche et il est chauffé à l'étape de chauffage (b) dans ledit contenant dans un état de fermeture étanche.

5. Procédé selon la revendication 4, dans lequel à l'étape de fourniture (a), le contenant étanche est mis dans un sachet scellé et il est chauffé à l'étape de chauffage (b) dans ledit sachet scellé.

6. Procédé selon l'une quelconque des revendications précédentes, dans lequel l'étape de chauffage (b) est réalisée à la vapeur d'eau, au bain marie, ou à la chaleur sèche.

7. Procédé selon l'une quelconque des revendications précédentes, dans lequel à l'étape de fourniture (a), les minéraux représentent entre 20 % et 60 % en poids sec du produit granulaire et le collagène représente entre 40 % et 80 % en poids sec du produit granulaire.

8. Procédé selon l'une quelconque des revendications précédentes, dans lequel à l'étape de fourniture (a), la solution aqueuse du mélange de départ est choisie parmi l'eau, un sérum physiologique ou une solution de tampon physiologique.

9. Procédé selon l'une quelconque des revendications précédentes, dans lequel à l'étape de fourniture (a), la solution aqueuse représente entre 40 % et 75 % en poids total du mélange de départ.

10. Procédé selon l'une quelconque des revendications précédentes, dans lequel à l'étape de fourniture (a), le matériau granulaire a une granulométrie inférieure à 4 mm, préférentiellement inférieure à 1,5 mm.

11. Procédé selon l'une quelconque des revendications précédentes, dans lequel à l'étape de fourniture (a), le mélange de départ comporte en outre un biomatériau ostéoconducteur minéralisé.

12. Procédé selon l'une quelconque des revendications précédentes, dans lequel à l'étape de fourniture (a), le mélange de départ comporte des grains de matrice osseuse totalement déminéralisée et des grains de matrice osseuse minéralisée.

13. Procédé selon l'une quelconque des revendications 1 à 11, dans lequel à l'étape de fourniture (a), le mélange de départ comporte des grains de matrice osseuse partiellement déminéralisée comprenant entre 20 % et 70 % de minéraux en poids sec, préférentiellement entre 40 % et 60 % de minéraux en poids sec.

14. Procédé selon l'une quelconque des revendications précédentes, dans lequel à l'étape de fourniture (a), le mélange de départ comprend du collagène purifié et des grains de matrice osseuse minéralisée.

15. Procédé selon la revendication 12, dans lequel l'étape de fourniture (a) comprend au moins les sous-étapes suivantes :
(a1) une sous-étape de préparation de grains de matrice osseuse totalement déminéralisée par déminéralisation et broyage à partir d'os,
(a2) une sous-étape de préparation de grains de matrice osseuse minéralisée par broyage d'os,
(a3) une sous-étape de mélangeage au cours de laquelle on mélange au moins les grains de matrice osseuse totalement déminéralisée, les grains de matrice osseuse minéralisée et la solution aqueuse pour obtenir ledit mélange de départ.

16. Procédé selon la revendication 13, dans lequel l'étape de fourniture (a) comprend au moins les sous-étapes suivantes :
(a'1) une sous-étape de préparation de grains de matrice osseuse partiellement déminéralisée par déminéralisation et broyage d'os,
(a'3) une sous-étape de mélangeage au cours de laquelle on mélange au moins les grains de matrice osseuse partiellement déminéralisée et la solution aqueuse pour obtenir ledit mélange de départ.

17. Procédé selon la revendication 14, dans lequel l'étape de fourniture (a) comprend au moins les sous-étapes suivantes :
(a"2) une sous-étape de préparation de grains de matrice osseuse minéralisée par broyage d'os,
(a"3) une sous-étape de mélangeage au cours de laquelle on mélange au moins les grains de matrice osseuse minéralisée, le collagène purifié et la solution aqueuse pour obtenir ledit mélange de départ.

18. Procédé selon la revendication 15 ou la revendication 16 ou la revendication 17, dans lequel au cours de la sous-étape de mélangeage, on mélange au moins un biomatériau ostéoconducteur minéralisé avec les autres constituants du mélange de départ.

## Patentansprüche

1. Verfahren zur Herstellung einer osteokonduktiven Knochenpaste, umfassend mindestens die folgenden Schritte:
(a) einen Schritt des Bereitstellens, in dem in einem Behälter (2, 3) ein Ausgangsgemisch bereitgestellt wird, das eine wässrige Lösung und ein Granulaterzeugnis, das mindestens Kollagen und Mineralien umfasst, enthält, wobei das Kollagen zwischen 30 und 90% Trockengewicht des Granulaterzeugnisses darstellt und die Mineralien zwischen 10 und 70% Trockengewicht des Granulaterzeugnisses darstellen, wobei mindestens ein Teil des Kollagens in den Granula der zumindest teilweise mineralisierten Knochenmatrix enthalten ist,
(b) einen Erwärmungsschritt, in dem in dem Behälter (2, 3) das Ausgangsgemisch auf eine Temperatur von mindestens 100°C, bevorzugt von 121°C erwärmt wird, um einen Teil von Kollagen in Gelatine umzuwandeln, sodass eine Kontinuität zwischen der Gelatine und der zumindest teilweise mineralisierten Knochenmatrix hergestellt wird und sodass dadurch eine kohäsive Knochenpaste bestimmter Viskosität erhalten wird, wobei der Erwärmungsschritt die Sterilisierung der Knochenpaste in dem Behälter (2, 3) ermöglicht,
wodurch die Knochenpaste (4) nach dem Abkühlen und dem Gelieren gebrauchsfertig ist und in dem Behälter (2, 3) in einem Dichtungszustand des Behälters steril gehalten wird.

2. Verfahren nach Anspruch 1, wobei in dem Erwärmungsschritt (b) das Ausgangsgemisch für einen Zeitraum zwischen 10 Minuten und 2 Stunden, bevorzugt zwischen 18 Minuten und 1 Stunde, erhitzt wird.

3. Verfahren nach Anspruch 1 oder 2, wobei in dem Erwärmungsschritt (b) das Ausgangsgemisch für einen Zeitraum zwischen 18 Minuten und 1 Stunde und auf eine Temperatur zwischen 100°C und 150°C erhitzt wird.

4. Verfahren nach einem der vorangehenden Ansprüche, wobei in dem Schritt des Bereitstellens (a) das Ausgangsgemisch in dem Behälter in einem Dichtungszustand bereitgestellt wird und in dem Erwärmungsschritt (b) in dem Behälter in einem Dichtungszustand erhitzt wird.

5. Verfahren nach Anspruch 4, wobei in dem Schritt des Bereitstellens (a) der dichte Behälter in einen versiegelten Beutel gegeben wird und in dem Erwärmungsschritt (b) in dem versiegelten Beutel erhitzt wird.

6. Verfahren nach einem der vorangehenden Ansprüche, wobei der Erwärmungsschritt (b) mit Wasserdampf, im Wasserbad oder bei trockener Hitze durchgeführt wird.

7. Verfahren nach einem der vorangehenden Ansprüche, wobei die Mineralien in dem Schritt des Bereitstellens (a) zwischen 20% und 60% Trockengewicht des Granulaterzeugnisses darstellen und das Kollagen zwischen 40% und 80% Trockengewicht des Granulaterzeugnisses darstellt.

8. Verfahren nach einem der vorangehenden Ansprüche, wobei in dem Schritt des Bereitstellens (a) die wässrige Lösung des Ausgangsgemisches ausgewählt ist aus Wasser, einer Kochsalzlösung oder einer physiologischen Pufferlösung.

9. Verfahren nach einem der vorangehenden Ansprüche, wobei in dem Schritt des Bereitstellens (a) die wässrige Lösung zwischen 40% und 75% Gesamtgewicht des Ausgangsgemisches darstellt.

10. Verfahren nach einem der vorangehenden Ansprüche, wobei in dem Schritt des Bereitstellens (a) das Granulatmaterial eine Partikelgröße von weniger als 4 mm, bevorzugt von weniger als 1,5 mm, aufweist.

11. Verfahren nach einem der vorangehenden Ansprüche, wobei in dem Schritt des Bereitstellens (a) das Ausgangsgemisch des Weiteren ein mineralisiertes osteokonduktives Biomaterial umfasst.

12. Verfahren nach einem der vorangehenden Ansprüche, wobei in dem Schritt des Bereitstellens (a) das Ausgangsgemisch Granula der vollständig entmineralisierten Knochenmatrix und Granula der mineralisieren Knochenmatrix umfasst.

13. Verfahren nach einem der vorangehenden Ansprüche 1 bis 11, wobei in dem Schritt des Bereitstellens (a) das Ausgangsgemisch Granula der teilweise entmineralisierten Knochenmatrix umfasst, die zwischen 20% und 70% Trockengewicht an Mineralien, bevorzugt zwischen 40% und 60% Trockengewicht an Mineralien, umfasst.

14. Verfahren nach einem der vorangehenden Ansprüche, wobei in dem Schritt des Bereitstellens (a) das Ausgangsgemisch aufgereinigtes Kollagen und Granula der mineralisierten Knochenmatrix umfasst.

15. Verfahren nach Anspruch 12, wobei der Schritt des Bereitstellens (a) mindestens die folgenden Unterschritte umfasst:
(a1) einen Unterschritt der Herstellung von Granula vollständig entmineralisierter Knochenmatrix durch Entmineralisierung und Mahlen von Knochen,
(a2) einen Unterschritt zur Herstellung von Granula mineralisierter Knochenmatrix durch Mahlen von Knochen,
(a3) einen Unterschritt des Mischens, in dem mindestens die Granula der vollständig entmineralisierten Knochenmatrix, die Granula der mineralisierten Knochenmatrix und die wässrige Lösung gemischt werden, um das Ausgangsgemisch zur erhalten.

16. Verfahren nach Anspruch 13, wobei der Schritt des Bereitstellens (a) mindestens die folgenden Unterschritte umfasst:
(a'1) einen Unterschritt zur Herstellung von Granula der teilweise entmineralisierten Knochenpaste durch Entmineralisierung und durch Mahlen von Knochen,
(a'3) einen Unterschritt des Mischens, in dem mindestens die Granula der teilweise entmineralisierten Knochenmatrix und die wässrige Lösung gemischt werden, um das Ausgangsgemisch zu erhalten.

17. Verfahren nach Anspruch 14, wobei der Schritt des Bereitstellens (a) mindestens die folgenden Unterschritte umfasst:
(a"2) einen Unterschritt zur Herstellung von Granula der mineralisierten Knochenmatrix durch Mahlen von Knochen,
(a"3) einen Unterschritt des Mischens, in dem mindestens die Granula der mineralisierten Knochenmatrix, das gereinigte Kollagen und die wässrige Lösung gemischt werden, um das Ausgangsgemisch zu erhalten.

18. Verfahren nach Anspruch 15 oder Anspruch 16 oder Anspruch 17, wobei in dem Unterschritt des Mischens mindestens ein mineralisiertes osteokonduktives Biomaterial mit den anderen Bestandteilen des Ausgangsgemisches gemischt wird.

## Claims

1. Method for preparing an osteoconductive bone paste, comprising at least the following steps:
(a) a providing step during which an initial mixture is supplied, in a container (2, 3), including an aqueous solution and a granular product comprising at least collagen and minerals, the collagen representing between 30 and 90% of the granular product by dry weight and the minerals representing between 10 and 70% of the granular product by dry weight, at least a portion of the collagen being contained in particles of at least partially mineralized bone matrix,
(b) a heating step during which the initial mixture is heated in the container (2, 3), at a temperature of at least 100°C, preferably 121 °C, to transform a portion of the collagen into gelatin so that there is continuity between the gelatin and the at least partially mineralized bone matrix and a cohesive bone paste is thus obtained of defined viscosity, the heating step enabling sterilization of the bone paste in the container (2, 3),
by means of which, after cooling and gelling, the bone paste (4) is ready for use and remains sterile in said container (2, 3), the container being in a closed and fluidtight state.

2. Method according to claim 1, wherein, in the heating step (b), the initial mixture is heated for a period of between 10 minutes and 2 hours, preferably between 18 min and 1 hour.

3. Method according to claim 1 or 2, wherein, in the heating step (b), the initial mixture is heated for a period of between 18 minutes and 1 hour and at a temperature of between 100°C and 150°C.

4. Method according to any preceding claim, wherein, in the providing step (a), the initial mixture is supplied in the container in a closed and fluidtight state, and it is heated in the heating step (b) in said container in this closed and fluidtight state.

5. Method according to claim 4, wherein, in the providing step (a), the fluidtight container is placed in a sealed pouch and is heated in the heating step (b) in said sealed pouch.

6. Method according to any preceding claim, wherein the heating step (b) is carried out in water vapor, a water bath, or dry heat.

7. Method according to any preceding claim, wherein, in the providing step (a), the minerals represent between 20% and 60% by dry weight of the granular product and the collagen represents between 40% and 80% by dry weight of the granular product.

8. Method according to any preceding claim, wherein, in the providing step (a), the aqueous solution of the initial mixture is selected from among water, normal saline, or a physiological buffer solution.

9. Method according to any preceding claim, wherein, in the providing step (a), the aqueous solution represents between 40% and 75% of the initial mixture by total weight.

10. Method according to any preceding claim, wherein, in the providing step (a), the granular product has a particle size of less than 4 mm, preferably less than 1.5 mm.

11. Method according to any preceding claim, wherein, in the providing step (a), the initial mixture further contains a mineralized osteoconductive biomaterial.

12. Method according to any preceding claim, wherein, in the providing step (a), the initial mixture contains completely demineralized bone matrix particles and mineralized bone matrix particles.

13. Method according to any one of claims 1 to 11, wherein, in the providing step (a), the initial mixture contains partially demineralized bone matrix particles comprising between 20% and 70% minerals by dry weight, preferably between 40% and 60% minerals by dry weight.

14. Method according to any preceding claim, wherein, in the providing step (a), the initial mixture comprises purified collagen and mineralized bone matrix particles.

15. Method according to claim 12, wherein the providing step (a) comprises at least the following sub-steps:
(a1) a sub-step of preparing completely demineralized bone matrix particles by demineralization and grinding of bone,
(a2) a sub-step of preparing mineralized bone matrix particles by grinding bone,
(a3) a sub-step of mixing during which at least the completely demineralized bone matrix particles, the mineralized bone matrix particles, and the aqueous solution are mixed to obtain said initial mixture.

16. Method according to claim 13, wherein the providing step (a) comprises at least the following sub-steps:
(a'1) a sub-step of preparing partially demineralized bone matrix particles by demineralization and grinding of bone,
(a'3) a sub-step of mixing during which at least the partially demineralized bone matrix particles and the aqueous solution are mixed to obtain said initial mixture.

17. Method according to claim 14, wherein the providing step (a) comprises at least the following sub-steps:
(a"2) a sub-step of preparing mineralized bone matrix particles by grinding bone,
(a"3) a sub-step of mixing during which at least the mineralized bone matrix particles, the purified collagen, and the aqueous solution are mixed to obtain said initial mixture.

18. Method according to claim 15 or claim 16 or claim 17, wherein, during the mixing sub-step, at least one mineralized osteoconductive biomaterial is mixed with the other constituents of the initial mixture.
